# EUROPÄISCHE PATENTANMELDUNG

(11) **EP 3 037 076 A1**
(43) Veröffentlichungstag der Anmeldung: **29.06.2016**
(21) Anmeldenummer: 15200351.3
(22) Anmeldetag: 16.12.2015
(51) Int. Cl.: A61F 7/08, A61N 5/06, A61N 2/02, A61F 7/00

(54) **MAGNETFELDRESONANZSYSTEM**

(30) Priorität: 23.12.2014 AT 5020314 U
(71) Anmelder: Geoway life balance GmbH, 8430 Leibnitz (AT)
(72) Erfinder: HILGARTH, Kurt, 8430 Leibnitz (AT)
(74) Vertreter: Schwarz & Partner

(57) **Zusammenfassung**

Die Erfindung betrifft eine Magnetfeldvorrichtung (100) zur therapeutischen Behandlung eines Benutzers (400), umfassend eine Magnetfeldmatte (110) mit einer Mattenoberseite (111), einer Mattenunterseite (112) sowie mit mehreren dazwischen angeordneten Magnetspulen (120), die von einem gepulsten elektrischen Strom durchfließbar sind, wobei jede Magnetspule (120) an zumindest zwei einander gegenüberliegende weichmagnetische Einsatzkörper (125) angrenzt, welche flächig zwischen der Mattenoberseite (111) und der Mattenunterseite (112) angeordnet sind. Weiters wird im Rahmen der Erfindung ein Magnetfeldresonanzsystem (1) umfassend eine Magnetfeldvorrichtung (100) angegeben, wobei von einer Steuervorrichtung (10) aus die Magnetfeldvorrichtung (100) sowie eine Lichttherapievorrichtung (200) und/oder eine Infrarottherapievorrichtung (300) gemeinsam steuerbar sind.

## Beschreibung

Die Erfindung betrifft eine Magnetfeldvorrichtung mit den Merkmalen des Oberbegriffs von Anspruch 1. Weiters wird im Rahmen der Erfindung ein Magnetfeldresonanzsystem, welches eine Magnetfeldvorrichtung umfasst, angegeben.

Unter einem Magnetfeldresonanzsystem wird insbesondere eine Einrichtung verstanden, mittels welcher eine Magnetresonanz-Therapie durchführbar ist, mit der also ein Benutzer mit elektromagnetischen Feldern gezielt behandelt werden kann. Ausschlaggebend für die Art der Wirkung oder auch der Schädigung von elektromagnetischen Wellen sind dabei Frequenz und Stärke des magnetischen Feldes.

Bekanntlich sind die meisten Funktionen des menschlichen Körpers rhythmische Prozesse. Dabei hat jedes Organsystem einen gewissen Grundrhythmus, der aus den Frequenzen seiner Einzelteile zusammengesetzt ist. Durch diese biologischen Rhythmen wird ein wesentlicher Beitrag zur inneren Optimierung und Kontrolle der Gesunderhaltung jedes Menschen erreicht. Die Anwendung von pulsierenden Magnetfeldern in den entsprechenden Frequenzen und mit den entsprechenden magnetischen Feldstärken kann dabei gezielt Körper- und Gehirnfunktionen unterstützen und zur Energiesteigerung, zur Aktivierung der Selbstheilungskräfte sowie zur Stressbewältigung und Regeneration eines Benutzers nach körperlicher Anstrengung führen.

Zur Behandlung kommen meist Magnetfeldmatten zum Einsatz, die in verschiedenen Größen erhältlich sind. Dies hängt davon ab, ob sie für die Behandlung des ganzen Körpers oder einzelner Körperteile konzipiert sind. Sowohl Ganz- als auch Teilkörpermatten enthalten Spulen, durch die gepulste niederfrequente elektrische Ströme fließen. Diese Ströme erzeugen Magnetfelder, die den Körper der behandelten Person durchdringen. Diese Magnetfelder erzeugen im elektrisch leitenden Körper eines Benutzers wiederum elektrische Ströme, die bei ausreichender Intensität physiologische Effekte hervorrufen können.

Signalform, Frequenz, Magnetfeldstärke und Spulenanordnung unterscheiden sich je nach Hersteller der Matten. Die Spulen weisen beispielsweise entweder eine spiralförmige Form auf oder sind in mehrere unterschiedliche starke Spulenpaare aufgeteilt.

Nachteilig an den bisher aus dem Stand der Technik bekannten Magnetfeldmatten ist meist, dass das von solchen Magnetfeldmatten erzeugte Magnetfeld meist heterogen aufgebaut ist und lokal unterschiedliche Magnetfeldstärken aufweist. Beispielsweise können bei Liegematten, also Magnetfeldmatten mit einem größerem Flächenmaß, die so dimensioniert sind, dass sich ein Benutzer während der Behandlung mit seinem ganzen Körper oder zumindest mit einem Körperabschnitt auf die Liegematte legt, besonders in der Mitte der Liegematte besonders hohe Magnetfeldstärken bzw. hohe Belastungen für den Körper des Benutzers auftreten. Weiters ist zu beachten, dass Benutzer während der Behandlung nicht immer korrekt auf einer Magnetfeldmatte positioniert sind, wodurch es ebenfalls zu erhöhten Körperströmen beispielsweise in peripheren Körperbereichen wie der Achsel- oder Leistengegend eines Benutzers kommt. Dabei kann es beim Einsatz von herkömmlichen Magnetfeldmatten auch zu einer unerwünschten lokalen Überschreitung von Grenzwerten einer maximalen Magnetfeldbelastung bzw. einer maximalen Magnetflussdichte kommen, wodurch gesundheitliche Beeinträchtigungen insbesondere bei längerem Gebrauch solcher Magnetfeldmatten nicht ausgeschlossen werden können.

Es ist somit Aufgabe der vorliegenden Erfindung eine Magnetfeldvorrichtung bereitzustellen, die die geschilderten Nachteile des Standes der Technik vermeidet und die im Betrieb ein besonders homogenes, flächiges Magnetfeld ausbildet.

Diese Aufgabe wird bei einer Magnetfeldvorrichtung gemäß dem Oberbegriff des Anspruchs 1 mit den Merkmalen des kennzeichnenden Teiles des Anspruchs 1 gelöst. Vorteilhafte Ausgestaltungen und Fortbildungen der Erfindung sind in den Unteransprüchen und der Beschreibung dargelegt.

Bei einer erfindungsgemäßen Magnetfeldvorrichtung zur therapeutischen Behandlung eines Benutzers, umfassend eine Magnetfeldmatte mit einer Mattenoberseite, einer Mattenunterseite sowie mit mehreren dazwischen angeordneten Magnetspulen, die von einem gepulsten elektrischen Strom durchfließbar sind, grenzt jede Magnetspule an zumindest zwei einander gegenüberliegende weichmagnetische Einsatzkörper an, welche flächig zwischen der Mattenoberseite und der Mattenunterseite angeordnet sind. Vorteilhaft wird durch den Einsatz der weichmagnetischen Einsatzkörper, die an die Magnetspulen angrenzen, während des Betriebs der Magnetfeldvorrichtung, wenn also die Magnetspulen stromdurchflossen sind, das dabei gebildete Magnetfeld vergleichmäßigt, eine magnetische Flussdichte des Magnetfelds erhöht und ein üblicherweise heterogenes magnetisches Streufeld dadurch homogener.

Als weichmagnetische Werkstoffe werden ferromagnetische Materialien bezeichnet, die sich in einem Magnetfeld leicht magnetisieren lassen. Eine magnetische Polarisation kann beispielsweise durch einen elektrischen Strom in einer stromdurchflossenen Spule - wie dies hier bei der Magnetfeldmatte der Fall ist - oder durch Anwesenheit eines Permanentmagneten erzeugt werden. Die Polarisation führt in allen weichmagnetischen Werkstoffen zu einer vielfach höheren magnetischen Flussdichte, als sie das von außen wirkende magnetische Feld in Luft erzeugt. Vereinfacht ausgedrückt wird durch den Einsatz eines weichmagnetischen Materials in einem Magnetfeld ein äußeres Magnetfeld um die Werkstoffpermeabilität des weichmagnetischen Materials verstärkt bzw. erhöht.

Im Gegensatz zu hartmagnetischen Werkstoffen, dies sind beispielsweise Dauermagneten, wird bei weichmagnetischen Stoffen ein Hystereseverlust beim Ummagnetisieren klein gehalten. Da neben dem Hystereseverlust auch ein Wirbelstromverlust verringert werden soll, werden bei netztypischen Frequenzen beispielsweise bei Eisenlegierungen widerstandserhöhende Legierungszusätze wie Silizium und Aluminium verwendet. Bei hohen Frequenzen werden wenig oder nichtleitende Ferrite als weichmagnetische Werkstoffe eingesetzt. Generell können als Werkstoffe für weichmagnetische Einsatzkörper Metalle in massiver Form, als gebundene Pulver, in Form von Blechen, kristallinen oder amorphen Bändern, oder auch als Drähte eingesetzt werden. Ebenso können ferrimagnetische keramische Werkstoffe, vor allem Ferrite auf Basis von Metalloxiden, für weichmagnetische Einsatzkörper eingesetzt werden.

Weitverbreitete Bauformen weichmagnetischer Werkstoffe sind beispielsweise: Kernbleche, Ringkerne, Schnittbandkerne, geklebte Blechpakete, Form- und Massivteile, Pulverkerne, geteilte bzw. schalenförmige Kernformen bei der Verwendung von ferrimagnetischen keramischen Werkstoffen, dünne Schichten sowie Drähte.

Zweckmäßig sind bei einer erfindungsgemäßen Magnetfeldvorrichtung die weichmagnetischen Einsatzkörper in der Magnetfeldmatte reihenförmig parallel zueinander angeordnet. Der Abstand zweier zueinander parallel angeordneter weichmagnetischer Einsatzkörper entspricht dabei der Größe der dazwischen befindlichen stromdurchflossenen Magnetspulen, die an zwei benachbarte weichmagnetische Einsatzkörper angrenzen. Wobei die Größe der flächigen Magnetspulen in Richtung der Längsseite und/oder der Schmalseite der Magnetfeldmatte gemessen wird. Je nach Ausführung ist bzw. sind beispielsweise eine oder mehrere Magnetspulen alternierend in jeder zweiten Reihe bzw. in jedem zweiten Zwischenraum zweier reihenförmig parallel zueinander angeordneter Einsatzkörper vorgesehen. In diesem Fall ist bzw. sind eine oder mehrere Magnetspulen in einem ersten Zwischenraum zwischen zwei reihenförmig parallel zueinander angeordneten Einsatzkörper innerhalb der Magnetfeldmatte befestigt. Ein daran angrenzender nächster bzw. zweiter Zwischenraum einer weiteren Reihe zweier Einsatzkörper bleibt frei und es sind hier keine Magnetspulen vorgesehen. Weitere Magnetspulen sind erst wieder in einem daran angrenzenden, dritten Zwischenraum einer weiteren Reihe zweier Einsatzkörper vorgesehen.

Oder alternativ dazu können im Rahmen der Erfindung beispielsweise in jedem Zwischenraum zwischen zwei reihenförmig parallel zueinander angeordneten Einsatzkörper eine oder mehrere Magnetspulen vorgesehen sein.

Zweckmäßig sind bei einer Magnetfeldvorrichtung gemäß der Erfindung die weichmagnetischen Einsatzkörper quer zu einer Längsseitenfläche der Magnetfeldmatte angeordnet. In dieser Ausführungsvariante wird eine besonders gleichmäßige Verteilung bzw. Anordnung der Einsatzkörper sowie der dazwischen vorgesehenen Magnetspulen erreicht. Die Einsatzkörper sind beispielsweise bei einer im Wesentlichen rechteckigen Magnetfeldmatte jeweils quer zu einer Längsseitenfläche angeordnet und erstrecken sich somit reihenweise voneinander beabstandet jeweils in Richtung der Mattenbreite zwischen den Längsseitenflächen der Magnetfeldmatte. Somit lässt sich die Magnetfeldmatte auch in ihrer Längsrichtung falten bzw. einrollen, was einen weiteren Vorteil dieser Ausführung darstellt. Ebenso kann es jedoch je nach Größe und Anwendungsgebiet der Magnetfeldmatte vorteilhaft sein die weichmagnetischen Einsatzkörper schräg oder auch bogenförmig zu einer Längsseitenfläche der Magnetfeldmatte anzuordnen.

In einer besonders vorteilhaften Ausführung der Erfindung sind bei einer Magnetfeldvorrichtung die weichmagnetischen Einsatzkörper aus flexibel biegbaren Streifen eines weichmagnetischen Werkstoffs gebildet. Eine Magnetfeldmatte, in der die streifenförmigen, flexiblen Einsatzkörper integriert sind, kann besonders vielseitig eingesetzt werden, da sie sich auch an Unebenheiten eines Untergrunds anpasst und einfach und komfortabel in einer Richtung quer zu den Einsatzkörperstreifen gefaltet oder eingerollt werden kann.

Besonders zweckmäßig weist in einer bevorzugten Ausführung der Erfindung bei einer Magnetfeldvorrichtung die Magnetfeldmatte einen Kopfabschnitt mit einer geringeren Anzahl an Magnetspulen zwischen benachbarten weichmagnetischen Einsatzkörper sowie einen Rumpfabschnitt mit einer höheren Anzahl an Magnetspulen zwischen benachbarten weichmagnetischen Einsatzkörper auf. In dieser Ausführungsvariante, die besonders aus therapeutischer Sicht Vorteile bietet, ist eine Dichte der Magnetspulen und somit während des Betriebs der Magnetfeldvorrichtung auch eine damit erzeugbare Magnetflussdichte im Kopfabschnitt niedriger als in einem Rumpfabschnitt der Magnetfeldmatte. Somit kann bei zweckgemäßem Gebrauch der Magnetfeldmatte, wenn sich also eine zu behandelnde Person so auf die Matte legt, dass während der Behandlung der Kopf der Person im Kopfabschnitt der Magnetfeldmatte ruht und sich der Körper bzw. die Gliedmaßen der Person im Rumpfabschnitt mit einer höheren Dichte an Magnetspulen befinden, ein besonders wirkungsvoller Einsatz der Magnetfeldvorrichtung erzielt werden. Vorteilhaft ist das auf den Kopf der Person dabei einwirkende Magnetfeld schwächer als das auf den Rumpf bzw. die Gliedmaßen der Person einwirkende Magnetfeld.

In einer besonders kompakten Weiterbildung der Erfindung ist bei einer Magnetfeldvorrichtung zumindest eine Erwärmungseinrichtung vorgesehen, welche Heizdrähte, vorzugsweise Infrarotheizdrähte, umfasst, die innerhalb der Magnetfeldmatte zumindest abschnittsweise zwischen der Mattenoberseite und der Mattenunterseite angeordnet sind. In dieser besonders vorteilhaften Ausführung der Erfindung kann neben der positiven Wirkung des erzeugten Magnetfelds, das den Körper einer behandelten Person durchdringt, wobei im elektrisch leitenden Körper eines Benutzers wiederum elektrische Ströme ausgelöst werden, die bei ausreichender Intensität physiologische Effekte hervorrufen können, zusätzlich durch den Einsatz der Erwärmungseinrichtung eine Wärmetherapie durchgeführt werden. Dabei wird die Magnetfeldmatte mittels der Heizdrähte in ihrem Inneren von innen heraus erwärmt. Der Benutzer nimmt dabei die innere Strahlungswärme, insbesondere bei der Verwendung von Infrarotheizdrähten, die erzeugte Infrarotstrahlungswärme, als besonders angenehm während der Behandlung war.

Vorteilhaft wird bzw. werden bei einer erfindungsgemäßen Magnetfeldvorrichtung für die Magnetfeldmatte ein wärmeleitendes Bezugsmaterial und/oder ein wärmeleitendes Füllmaterial verwendet. Dadurch wird insbesondere bei einer Magnetfeldvorrichtung mit einer Erwärmungseinrichtung die positive Wirkung der Wärmetherapie während der Behandlung im Magnetfeld noch weiter verbessert.

Besonders vorteilhaft enthält bzw. enthalten das Bezugsmaterial und/oder das Füllmaterial der Magnetfeldmatte Carbonfasern. Carbonfasern sind insbesondere beim Einsatz von Infrarotheizdrähten besonders vorteilhaft, da sie Infrarotstrahlung sehr gut leiten bzw. abgeben.

In einer Weiterbildung der Erfindung umfasst eine Magnetfeldvorrichtung weiterhin eine Steuervorrichtung zur Programmauswahl eines oder mehrerer Frequenzbänder des gepulsten elektrischen Stroms zur Erzeugung eines Magnetfelds, wobei die Frequenzbänder gemäß ihrem Frequenzmuster ausgewählt sind aus der Gruppe: Sinus-Wechselfeld, Rechteck, Dreieck, Sägezahn, Impuls-Signal mit ansteigender positiver Halbwelle, Multi-Resonanz. Im Rahmen der Erfindung können sämtliche derzeit bekannte Frequenzmuster ausgewählt werden. Je nach Alter, Gewicht und Konstitution der Benutzer können damit individuelle Anwendungsprogramme bei unterschiedlichen Ausgangsfrequenzen beispielsweise von 1 Hz bis 10.000 Hz sowie bei unterschiedlichen magnetischen Flussdichten erzielt werden. Ebenso ist es im Rahmen der Erfindung vorgesehen, die Magnetfeldvorrichtung sowohl im Dauerbetrieb, als auch zeitlich gesteuerten Programmarten, bei denen sich die Frequenzmuster und/oder die magnetischen Flussdichten während der Behandlung ändern, zu betreiben.

In einer Weiterbildung der Erfindung wird weiters ein Magnetfeldresonanzsystem angegeben, welches eine erfindungsgemäße Magnetfeldvorrichtung umfasst, wobei von einer Steuervorrichtung aus die Magnetfeldvorrichtung sowie eine Lichttherapievorrichtung umfassend zumindest eine Farblichtleuchte und/oder eine Wärmetherapievorrichtung, welche zumindest eine Infrarotlichtleuchte und/oder eine Erwärmungsvorrichtung der Magnetfeldvorrichtung umfasst, gemeinsam steuerbar sind. Vorteilhaft sind mit einem derartigen Magnetfeldresonanzsystem neben einer Magnetfeldtherapie weiters auch eine Lichttherapie und/oder eine Wärmetherapie gleichzeitig oder alternierend möglich. Durch die gemeinsame Steuervorrichtung können die einzelnen Therapieformen vorteilhaft aufeinander abgestimmt und gemeinsam gesteuert werden. Mit der Lichttherapievorrichtung, welche zumindest eine Farblichtleuchte umfasst, können die nachgewiesenen positiven Effekte einer Farblichtbehandlung mit der Magnetfeldbehandlung kombiniert werden. Zusätzlich oder alternativ dazu kann mit der Wärmetherapievorrichtung, welche zumindest eine Infrarotlichtleuchte und/oder eine Erwärmungsvorrichtung der Magnetfeldvorrichtung umfasst, auch eine vorteilhafte Wärmetherapie bei der zu behandelnden Person durchgeführt werden. Dazu ist beispielsweise die Magnetfeldmatte mit einer zuvor beschriebenen Erwärmungsvorrichtung ausgestattet bzw. an diese angeschlossen. Oder es ist zumindest eine Infrarotleuchte vorgesehen, mit der die zu behandelnde Person mit Infrarotlicht bestrahlt werden kann, während sie sich liegend auf der Magnetfeldmatte befindet. Selbstverständlich sind auch beliebige Kombinationen der zuvor beschriebenen Ausstattungen im Rahmen eines erfindungsgemäßen Magnetfeldresonanzsystems möglich.

Zweckmäßig ist bei einem Magnetfeldresonanzsystem die Steuervorrichtung mit einem Lichttherapiewahlschalter zur Auswahl unterschiedlicher Farbtemperaturen der zumindest einen Farblichtleuchte ausgestattet. Mit dem Lichttherapiewahlschalter können die Farbspektren bzw. Farbtemperaturen einer oder mehrerer Farblichtleuchten entsprechend den individuellen Wünschen des Benutzers eingestellt und ausgewählt werden.

In einer Weiterbildung der Erfindung ist bei einem Magnetfeldresonanzsystem zumindest eine Farblichtleuchte in der Steuervorrichtung integriert und/oder in einem mit der Steuervorrichtung verbundenen Leuchtengehäuse angeordnet. Besonders kompakt ist die zumindest eine Farblichtleuchte direkt in der Steuervorrichtung bzw. in deren Gehäuse integriert. Alternativ oder in Ergänzung dazu können eine oder mehrere Farblichtleuchten auch in einem separaten Leuchtengehäuse integriert sein. Das Leuchtengehäuse ist dabei beispielsweise mittels einer Steuersignalleitung und/oder einem Netzkabel mit der Steuervorrichtung verbunden und kann ebenfalls von dieser aus gesteuert werden.

In einer weiteren bevorzugten Ausführung ist bei einem Magnetfeldresonanzsystem die Steuervorrichtung mit einem Wärmetherapiewahlschalter zur Steuerung einer Erwärmungseinrichtung der Magnetfeldmatte und/oder der zumindest einen Infrarotlichtleuchte ausgestattet. Somit lassen sich zentral von der Steuervorrichtung aus auch Varianten einer Wärmetherapie auswählen.

Zweckmäßig ist die zumindest eine Infrarotlichtleuchte in einem mit der Steuervorrichtung verbundenen Leuchtengehäuse angeordnet. Das Leuchtengehäuse ist dabei beispielsweise mittels einer Steuersignalleitung und/oder einem Netzkabel mit der Steuervorrichtung verbunden und kann von dieser aus ebenfalls gesteuert werden. Das Leuchtengehäuse kann neben einer oder mehreren Infrarotlichtleuchten weiters auch zumindest eine Farblichtleuchte umfassen. Alternativ dazu kann im Rahmen eines erfindungsgemäßen Magnetfeldresonanzsystems ein eigenes Leuchtengehäuse, in dem ausschließlich eine oder mehrere Infrarotlichtleuchten installiert sind, zum Einsatz kommen.

Besonders zweckmäßig ist bzw. sind die Steuervorrichtung und/oder ein Leuchtengehäuse mit einem schwenkbaren Standfuß ausgerüstet. In dieser Ausführung können die entsprechend mit einem Standfuß ausgestatteten Einheiten eines Magnetfeldresonanzsystems besonders einfach aufgestellt werden. Durch den schwenkbaren Standfuß können beispielsweise Leuchten, die in der Steuervorrichtung bzw. im Leuchtengehäuse integriert sind, besonders komfortabel derart positioniert und ausgerichtet werden, dass die Lichtkegel auf die entsprechenden Körperpartien einer Person, die auf der Magnetfeldmatte liegt, gerichtet sind.

Weitere Einzelheiten, Merkmale und Vorteile der Erfindung ergeben sich aus der nachfolgenden Erläuterung eines in den Zeichnungen schematisch dargestellten Ausführungsbeispiels. In den Zeichnungen zeigen:
- Fig. 1 in einer Draufsicht eine Ausführungsvariante einer Steuervorrichtung eines erfindungsgemäßen Magnetfeldresonanzsystems;
- Fig. 2 eine schematische Ansicht eines erfindungsgemäßen Magnetfeldresonanzsystems umfassend eine Steuervorrichtung, eine Magnetfeldvorrichtung sowie eine kombinierte Lichttherapie- und Infrarottherapievorrichtung;
- Fig. 3 ein Frequenzmuster während einer Magnetfeldanwendung.

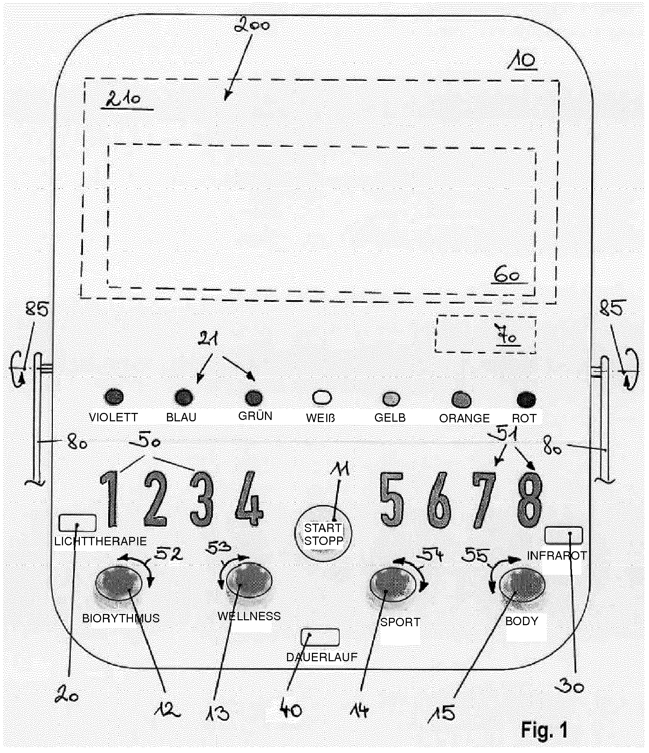

Fig. 1 betrifft eine mögliche Ausführung einer Steuervorrichtung 10 zur Steuerung eines erfindungsgemäßen Magnetfeldresonanzsystems 1. Die Steuervorrichtung 10 umfasst einen Ein-/Aus-Schalter 11, weiters Hauptprogrammwahlschalter 12 bzw. 13, 14, 15, mit denen unterschiedliche Hauptprogramme der Magnetfeldanwendung eingestellt werden können. Beispielsweise dienen die Hauptprogrammwahlschalter 12, 13, 14, 15 zur Auswahl von unterschiedlichen Magnetfeldanwendungsprogrammen mit den Bezeichnungen "Biorhythmus", "Wellness", "Sport" sowie "Body". Weiters ist ein Lichttherapiewahlschalter 20 vorgesehen, mit dem bedarfsweise zusätzlich zur Magnetfeldtherapie auch eine Lichttherapie aktiviert werden kann. Dazu weist die Steuervorrichtung 10 Lichttherapieanzeigen 21 auf, die beispielsweise hier vom Benutzer als eine von acht unterschiedlichen Farbtemperaturen einer multispektralen Farblichtleuchte ausgewählt werden können. Zusätzlich können mit einem Wärmetherapiewahlschalter 30 auch Varianten einer Wärmetherapie ausgewählt werden. Weiters ist ein Dauerbetriebsschalter 40 vorhanden, mit dem ein Dauerbetrieb des Magnetfeldresonanzsystems 1 und/oder ein Dauerbetrieb einzelner Therapieformen aktiviert werden kann.

Außerdem befinden sich Unterprogrammschalter 50 auf der Anzeige der Steuervorrichtung 10, die je nach Ausführung entweder als Unterprogrammanzeigen 51 dienen oder die selbst als Unterprogrammschalter 50 betätigt werden können und dazu beispielsweise als Tastsensoren ausgeführt sind. Alternativ oder in Ergänzung dazu können auch Unterprogrammwahlschalter 52 bzw. 53, 54, 55 jeweils in den Hauptprogrammwahlschalter 12 bzw. 13, 14, 15 integriert sein. Dabei wird beispielsweise ein Hauptprogramm durch Drücken eines der Hauptprogrammwahlschalter 12, 13, 14, 15 ausgewählt und danach ein entsprechendes Unterprogramm durch Drehen 52, 53, 54, 55 des zuvor entsprechend ausgewählten Hauptprogrammschalters festgelegt.

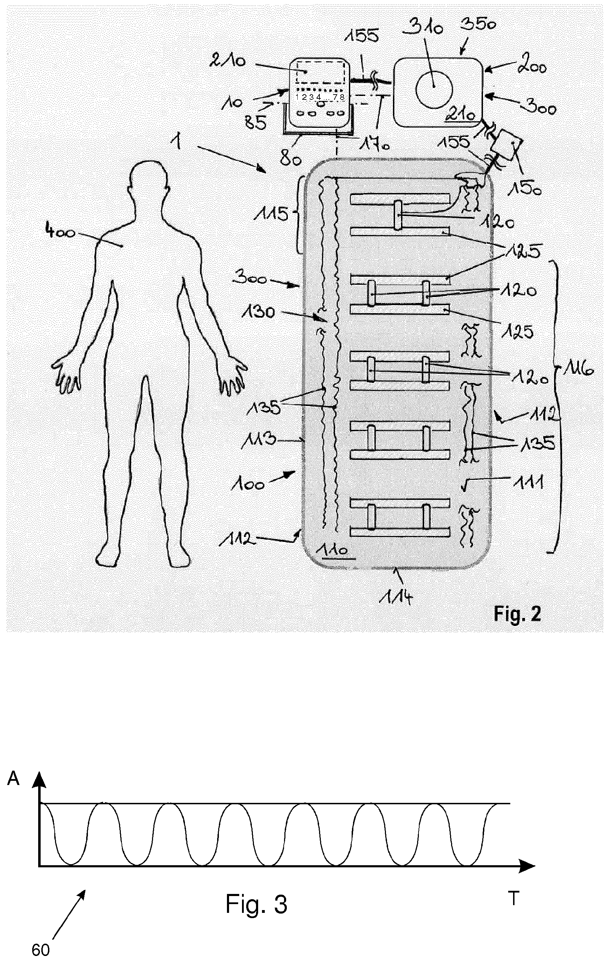

Weiters kann die Steuervorrichtung 10 beispielsweise ein optionales Anzeigefeld 60 umfassen, das hier in Fig. 1 ebenso wie ein mögliches Feld einer Fehleranzeige 70 strichliert eingezeichnet ist. Im Anzeigefeld 60 kann beispielsweise während der Magnetfeldanwendung ein aktuelles Frequenzmuster als Funktion eines Ausschlags A bzw. einer Amplitude A während einer Zeit T der Anwendung angezeigt werden, wie es in Fig. 3 veranschaulicht ist. Somit können die Amplitude A bzw. eine Periodendauer T unterschiedlicher Frequenzen während der Magnetfeldanwendungen visualisiert werden. Eine Fehleranzeige 70, die mögliche Fehlfunktionen der Steuervorrichtung 10 oder der mit ihr verbundenen Geräte visualisiert, kann ebenfalls an der Steuervorrichtung 10 vorgesehen sein.

Fig. 2 veranschaulicht ein erfindungsgemäßes Magnetfeldresonanzsystem 1 umfassend eine Steuervorrichtung 10, eine Magnetfeldvorrichtung 100 sowie eine kombinierte Lichttherapievorrichtung 200 und eine Wärmetherapievorrichtung 300. Die Magnetfeldvorrichtung 100 umfasst eine Magnetfeldmatte 110 mit einer Oberseite 111 sowie einer Unterseite 112. Die Magnetfeldmatte 110 ist hier im Wesentlichen als rechteckförmige, flache Matte mit Längsseitenflächen 113 entlang ihrer Mattenlänge sowie Schmalseitenflächen 114 entlang ihrer Mattenbreite gestaltet. Die Mattenlänge bzw. die Länge der Längsseitenflächen 113 entspricht hier etwa der Körpergröße einer erwachsenen Person bzw. eines Benutzers 400. Während der Magnetfeldresonanzanwendung liegt der Benutzer 400 auf der Magnetfeldmatte 110, wobei sein Kopf in einem Kopfabschnitt 115 und sein Rumpf bzw. seine Gliedmaßen in einem Rumpfabschnitt 116 der Magnetfeldmatte 110 aufliegen. Im Inneren der Magnetfeldmatte 110 sind dazu mehrere Magnetspulen 120 vorgesehen, die jeweils zwischen zwei weichmagnetischen Einsatzkörper 125 angeordnet sind. Im Bereich des Kopfabschnitts 115 ist die Dichte der Magnetspulen 120 geringer als im Rumpfabschnitt 116. Beispielhaft ist hier in Fig. 2 eine einzelne Magnetspule 120 im Kopfabschnitt 115 skizziert, die angrenzend an zwei weichmagnetische Einsatzkörper 125 angeordnet ist. Im Rumpfabschnitt 116 sind beispielsweise jeweils zwei Magnetspulen 120 nebeneinander vorgesehen, die jeweils an zwei weichmagnetische Einsatzkörper 125 angrenzen. Die weichmagnetischen Einsatzkörper 125 sind hier in Form von flachen Einsatzkörperstreifen reihenweise bzw. parallel zueinander in Längsrichtung der Magnetfeldmatte 110 voneinander beabstandet angeordnet. Stromkabel, die zur Stromversorgung der stromdurchflossenen Magnetspulen 120 dienen, sind hier zur besseren Übersicht hier nicht dargestellt. Weiters ist die Magnetfeldmatte 110 mit einer Erwärmungseinrichtung 130 ausgestattet. Dazu sind mehrere Infrarotheizdrähte 135 in Längsrichtung nebeneinander innerhalb der Magnetfeldmatte 110, also zwischen der Mattenoberseite 111 und der Mattenunterseite 112 angeordnet. Somit kann die Magnetfeldmatte 110 während der Magnetresonanzbehandlung zusätzlich auch von innen beheizt und als Wärmetherapievorrichtung 300 eingesetzt werden. Vorteilhaft sind hier ein Bezugsmaterial sowie ein Füllmaterial der Magnetfeldmatte 110 wärmeleitend ausgerüstet, wobei die verwendeten Bezugs- und Füllmaterialien besonders wärmeleitende Carbonfasern enthalten. Ein Netzgerät 150 dient zur Stromversorgung der Magnetfeldvorrichtung 1 und die zugehörigen einzelnen Geräte 10, 100, 200, 300 sind jeweils mittels mehrerer stromführender Netzkabel 155 mit dem Netzgerät 150 verbunden. Die Steuervorrichtung 10 ist mittels Steuersignalleitungen 170 mit der Magnetfeldmatte 110 sowie mit der Lichttherapievorrichtung 200 bzw. der Wärmetherapievorrichtung 300, die hier gemeinsam in einem Leuchtengehäuse 350 integriert sind, verbunden. Als Steuersignalleitungen 170 werden hier herkömmliche Signalleitungskabel verwendet. Im Rahmen der Erfindung ist es jedoch auch denkbar, Steuersignale von der Steuervorrichtung 10 mittels Funkübertragung kabellos an die entsprechenden peripheren Geräte 100, 200, 300 zu übertragen. Die Lichttherapievorrichtung 200 umfasst hier zwei Farblichtleuchten 210, wobei eine Farblichtleuchte 210 direkt in die Steuervorrichtung 10 integriert ist und eine weitere Farblichtleuchte 210 im separaten Leuchtengehäuse 350 vorgesehen ist. Zur Wärmetherapie umfasst die Wärmetherapievorrichtung 300 neben der Erwärmungsvorrichtung 130 innerhalb der Magnetfeldmatte 110 weiters eine Infrarotlichtleuchte 310, die ebenfalls im Leuchtengehäuse 350 integriert ist. Sowohl die Steuervorrichtung 10, als auch das Leuchtengehäuse 350 sind jeweils mit Standfüßen 80 ausgestattet, um eine komfortable und sichere Positionierung der Leuchten 210, 310 während der Magnetfeldbehandlung zu gewährleisten.

Weiters kann erwähnt werden, dass das die Steuervorrichtung des Magnetfeldresonanzsystems zum Steuern der Magnetfeldvorrichtung im Bereich des Rumpfabschnitts in einzelnen Körperzonen ausgebildet sein kann, um das Magnetfeld in einer Schulterzone, einer Hüftzone und/oder einer Kniezone unterschiedlich anzusteuern.

**Liste der Positionszeichen:**

| | | | |
|---|---|---|---|
| 1 | Magnetfeldresonanzsystem | 400 | Benutzer |
| 10 | Steuervorrichtung | A | Ausschlag, Amplitude |
| 11 | Ein-/Aus-Schalter | T | Zeit, Periodendauer |
| 12 | Hauptprogrammwahlschalter (bzw. 13, 14, 15) | | |
| 20 | Lichttherapiewahlschalter | | |
| 21 | Lichttherapieanzeige | | |
| 30 | Wärmetherapiewahlschalter | | |
| 40 | Dauerbetriebsschalter | | |
| 50 | Unterprogrammschalter | | |
| 51 | Unterprogrammanzeige | | |
| 52 | Unterprogrammwahlschalter (bzw. 53, 54, 55) | | |
| 60 | Anzeigefeld | | |
| 70 | Fehleranzeige | | |
| 80 | Standfuß bzw. Stativ | | |
| 85 | Schwenkachse | | |
| 100 | Magnetfeldvorrichtung | | |
| 110 | Magnetfeldmatte | | |
| 111 | Oberseite der Magnetfeldmatte | | |
| 112 | Unterseite der Magnetfeldmatte | | |
| 113 | Längsseitenfläche der Magnetfeldmatte | | |
| 114 | Schmalseitenfläche der Magnetfeldmatte | | |
| 115 | Kopfabschnitt | | |
| 116 | Rumpfabschnitt | | |
| 120 | Magnetspule | | |
| 125 | Weichmagnetischer Einsatzkörper | | |
| 130 | Erwärmungseinrichtung | | |
| 135 | Infrarotheizdraht | | |
| 150 | Netzgerät | | |
| 155 | Anschlusskabel | | |
| 170 | Steuersignalleitung | | |
| 200 | Lichttherapievorrichtung | | |
| 210 | Farblichtleuchte | | |
| 300 | Wärmetherapievorrichtung | | |
| 310 | Infrarotlichtleuchte | | |
| 350 | Leuchtengehäuse | | |

## Patentansprüche

1. Magnetfeldvorrichtung (100) zur therapeutischen Behandlung eines Benutzers (400), umfassend eine Magnetfeldmatte (110) mit einer Mattenoberseite (111), einer Mattenunterseite (112) sowie mit mehreren dazwischen angeordneten Magnetspulen (120), die von einem gepulsten elektrischen Strom durchfließbar sind, **dadurch gekennzeichnet, dass** jede Magnetspule (120) an zumindest zwei einander gegenüberliegende weichmagnetische Einsatzkörper (125) angrenzt, welche flächig zwischen der Mattenoberseite (111) und der Mattenunterseite (112) angeordnet sind.

2. Magnetfeldvorrichtung (100) nach Anspruch 1, **dadurch gekennzeichnet, dass** die weichmagnetischen Einsatzkörper (125) in der Magnetfeldmatte (110) reihenförmig parallel zueinander angeordnet sind.

3. Magnetfeldvorrichtung (100) nach Anspruch 1 oder 2, **dadurch gekennzeichnet, dass** die weichmagnetischen Einsatzkörper (125) quer und/oder schräg und/oder bogenförmig zu einer Längsseitenfläche (113) der Magnetfeldmatte (110) angeordnet sind.

4. Magnetfeldvorrichtung (100) nach einem der Ansprüche 1 bis 3, **dadurch gekennzeichnet, dass** die weichmagnetischen Einsatzkörper (125) aus flexibel biegbaren Streifen eines weichmagnetischen Werkstoffs gebildet sind.

5. Magnetfeldvorrichtung (100) nach einem der Ansprüche 1 bis 4, **dadurch gekennzeichnet, dass** die Magnetfeldmatte (110) einen Kopfabschnitt (115) mit einer geringeren Anzahl an Magnetspulen (120) zwischen benachbarten weichmagnetischen Einsatzkörper (125) sowie einen Rumpfabschnitt (116) mit einer höheren Anzahl an Magnetspulen (120) zwischen benachbarten weichmagnetischen Einsatzkörper (125) aufweist.

6. Magnetfeldvorrichtung (100) nach einem der Ansprüche 1 bis 5, **gekennzeichnet durch** zumindest eine Erwärmungseinrichtung (130) umfassend Heizdrähte, vorzugsweise Infrarotheizdrähte (135), die innerhalb der Magnetfeldmatte (110) zumindest abschnittsweise zwischen der Mattenoberseite (111) und der Mattenunterseite (112) angeordnet sind.

7. Magnetfeldvorrichtung (100) nach einem der Ansprüche 1 bis 6, **gekennzeichnet durch** ein wärmeleitendes Bezugsmaterial und/oder ein wärmeleitendes Füllmaterial der Magnetfeldmatte (110).

8. Magnetfeldvorrichtung (100) nach Anspruch 7, **dadurch gekennzeichnet, dass** das Bezugsmaterial und/oder das Füllmaterial Carbonfasern enthält bzw. enthalten.

9. Magnetfeldvorrichtung (100) nach einem der Ansprüche 1 bis 8, weiterhin umfassend eine Steuervorrichtung (10) zur Programmauswahl (12, 52) eines oder mehrerer Frequenzbänder des gepulsten elektrischen Stroms zur Erzeugung eines Magnetfelds, wobei die Frequenzbänder gemäß ihrem Frequenzmuster ausgewählt sind aus der Gruppe: Sinus-Wechselfeld, Rechteck, Dreieck, Sägezahn, Impuls-Signal mit ansteigender positiver Halbwelle, Multi-Resonanz.

10. Magnetfeldresonanzsystem (1) umfassend eine Magnetfeldvorrichtung (100) nach einem der Ansprüche 1 bis 9, **dadurch gekennzeichnet, dass** von einer Steuervorrichtung (10) aus die Magnetfeldvorrichtung (100) sowie eine Lichttherapievorrichtung (200) umfassend zumindest eine Farblichtleuchte (210) und/oder eine Wärmetherapievorrichtung (300), welche zumindest eine Infrarotlichtleuchte (310) und/oder eine Erwärmungsvorrichtung (130) der Magnetfeldvorrichtung (100) umfasst, gemeinsam steuerbar sind.

11. Magnetfeldresonanzsystem (1) nach Anspruch 10, **dadurch gekennzeichnet, dass** die Steuervorrichtung (10) mit einem Lichttherapiewahlschalter (20) zur Auswahl unterschiedlicher Farbtemperaturen der zumindest einen Farblichtleuchte (210) ausgestattet ist.

12. Magnetfeldresonanzsystem (1) nach Anspruch 10 oder 11, **dadurch gekennzeichnet, dass** zumindest eine Farblichtleuchte (210) in der Steuervorrichtung (10) integriert ist und/oder in einem mit der Steuervorrichtung (10) verbundenen (170) Leuchtengehäuse (350) angeordnet ist.

13. Magnetfeldresonanzsystem (1) nach einem der Ansprüche 10 bis 12, **dadurch gekennzeichnet, dass** die Steuervorrichtung (10) mit einem Wärmetherapiewahlschalter (30) zur Steuerung einer Erwärmungseinrichtung (130) der Magnetfeldmatte (110) und/oder der zumindest einen Infrarotlichtleuchte (310) ausgestattet ist.

14. Magnetfeldresonanzsystem (1) nach Anspruch 10 oder 13, **dadurch gekennzeichnet, dass** die zumindest eine Infrarotlichtleuchte (310) in einem mit der Steuervorrichtung (10) verbundenen (170) Leuchtengehäuse (350) angeordnet ist.

15. Magnetfeldresonanzsystem (1) nach einem der Ansprüche 10 bis 14, **dadurch gekennzeichnet, dass** die Steuervorrichtung (10) und/oder ein Leuchtengehäuse (350) mit einem schwenkbaren (85) Standfuß (80) ausgerüstet ist bzw. sind.
